# EUROPÄISCHE PATENTANMELDUNG

(11) **EP 1 031 568 A1**
(43) Veröffentlichungstag der Anmeldung: **30.08.2000**
(21) Anmeldenummer: 00102244.1
(22) Anmeldetag: 14.02.2000
(51) Int. Cl.: C07D 295/02

(54) **Verfahren zur Herstellung von cis-2,6-Dimethylpiperazin**

(30) Priorität: 24.02.1999 DE 19907829
(71) Anmelder: Bayer Aktiengesellschaft, 51368 Leverkusen (DE)
(72) Erfinder: Schubart, Rüdiger, Dr., 51467 Bergisch Gladbach (DE)

(57) **Zusammenfassung**

Es wird ein Verfahren zur selektiven Herstellung von cis-2,6-Dimethylpiperazin bereitgestellt, bei dem ein Diisopropanolamin-Gemisch, welches Verbindungen der Formel HN-(CH₂-CH(OH)CH₃)₂, HN-(CH(CH₃)-CH₂OH)₂ und HN(CH(CH₃)-CH₂OH)(CH₂-CH(OH)CH₃) enthält, oder 1,2-Diaminopropan mit Ammoniak und Wasserstoff in Gegenwart eines Hydrierkatalysators umgesetzt werden.

## Beschreibung

Die vorliegende Erfindung betrifft ein Verfahren zur Herstellung von cis-2,6-Dimethylpiperazin durch Umsetzung von Diisopropanolamin oder 1,2-Diaminopropan mit Ammoniak und Wasserstoff in Gegenwart eines Katalysators.

Substituierte Piperazine sind wertvolle Zwischenprodukte für die Synthese von Kautschukhilfsmitteln, insbesondere von Vulkanisationsbeschleunigern, sowie für die Synthese von Pflanzenschutzmitteln und pharmakologischen Wirkstoffen. Cis-2,6-Dimethylpiperazin ist z.B. ein wichtiger Ausgangsstoff für die Synthese von bestimmten antibakteriell wirksamen Chinoloncarbonsäurederivaten wie dem Sparfloxacin (Arzneim.-Forsch. 41 (1991), 744). Der Herstellung substituierter Piperazine, insbesondere des cis-2,6-Dimethylpiperazins, kommt daher steigende Bedeutung zu.

In der GB-A-1 295 784 wird ein Verfahren zur Herstellung von N-(2-Aminoethyl)-piperazin durch Cyclisierung von Aminoethanol in wäßrigem Medium in Gegenwart von Ammoniak, Wasserstoff und einem Katalysator beschrieben. Wesentlich ist bei der Durchführung dieses Verfahrens, daß ein Teil des Reaktionsgemisches nach destillativer Abtrennung einer möglichst großen Menge des gewünschten N-(2-Aminoethyl)-piperazins in die Umsetzung recycliert wird. Durch diese Maßnahme kann die Ausbeute an N-(2-Aminoethyl)-piperazin deutlich erhöht werden.

Aus der US-A-3,692,789 ist ebenfalls ein Verfahren zur Herstellung von N-(2-Aminoethyl)-piperazin bekannt. In Gegenwart von Ammoniak, Wasserstoff und einem Hydrierkatalysator wird N-(Hydroxyethyl)-diethylentriamin zu N-(2-Aminoethyl)-piperazin cyclisiert.

Die Synthese von Piperazin aus Aminoethanol wird gemäß US-A-3,112,318 in Gegenwart von Ammoniak, Wasserstoff und einem Hydrierkatalysator durchgeführt. Bei diesem Verfahren ist es wichtig, daß in Abwesenheit von Wasser gearbeitet wird.

Als Nebenprodukte werden bei der Umsetzung Ethylendiamin und N-(2-Amino-ethyl)-piperazin erhalten.

Im Gegensatz zu dem zuvor genannten Piperazin sowie den am Stickstoff substituierten Piperazinen weist 2,6-Dimethylpiperazin in 2- und 6-Stellung zwei asymmetrische Kohlenstoffatome auf, und es gibt somit zwei Stereoisomere, das trans- und cis-2,6-Dimethylpiperazin, die beide bei Synthesereaktionen prinzipiell entstehen können.

Aus der US-A-2,525,223 ist ein Verfahren zur Herstellung von Mono-N-Alkyl-Piperazinen durch Umsetzung von Dialkanolaminen in Form von Diethanolamin oder dessen Alkyl-Derivaten mit einem primären Alkylamin in Gegenwart eines Katalysators wie z.B. Raney-Nickel bekannt. Experimentell beschrieben werden ausschließlich Umsetzungen von Diethanolamin mit Methyl-, Ethyl- oder Isopropylamin zu Mono-N-(methyl-, ethyl- oder isopropyl)-piperazin. Die hierbei erzielten Umsätze sind sehr gering und liegen bei maximal 34%. Bezüglich des als Edukt prinzipiell genannten Di-(2-hydroxypropyl)-amins fehlen jegliche weitere Angaben, insbesondere darüber, welche Produkte und Stereoisomere bei der Umsetzung gebildet werden.

Aus JP-Laid Open Publication Hei 8-34773 ist ein Verfahren zur Herstellung von cis-2,6-Dimethylpiperazin ausgehend von Diisopropanolamin bekannt. Bei diesem Diisopropanolamin handelt es sich um reines Diisopropanolamin der Struktur HN-(CH₂-CH(OH)CH₃)₂ (Di-(2-hydroxypropyl)-amin), da im Reaktionsverlauf nur von der Bildung des cis- und trans-2,6-Dimethylpiperazins berichtet wird. Würde neben dem HN-(CH₂-CH(OH)CH₃)₂ auch noch HN(CH(CH₃)-CH₂OH)(CH₂CH(OH)CH₃) vorliegen, so hätte als Produkt der Cyclisierungsreaktion auch cis- und trans 2,5-Dimethylpiperazin entstehen müssen. Zunächst wird das reine, einheitliche Diisopropanolamin in einem organischen Lösungsmittel in Gegenwart eines Katalysators mit Ammoniak und Wasserstoff umgesetzt. Als organische Lösungsmittel werden dabei bevorzugt aromatische Kohlenwasserstoffe wie Benzol, Toluol oder Xylol verwendet. Das bei der Cyclisierung entstehende Gemisch aus cis-2,6- und trans-2,6-Dimethylpiperazin in dem organischen Lösungsmittel wird zunächst durch Filtration vom Katalysator abgetrennt, anschließend durch azeotrope Destillation vom Wasser befreit und nach weiterer Zugabe von organischem Lösungsmittel einer Kristallisation unterworfen. Das auskristallisierte cis-2,6-Dimethylpiperazin wird abgetrennt. Das im Reaktionsgemisch zurückbleibende trans-2,6-Dimethylpiperazin läßt sich anschließend in Gegenwart eines Katalysators bei einer Temperatur von mindestens 180°C teilweise zum cis-2,6-Dimethylpiperazin isomerisieren, wodurch die Gesamtausbeute zum cis-2,6-Dimethylpiperazin erhöht wird. Als entscheidend für die Selektivität der primären Cyclisierung zum cis-2,6-Dimethylpiperazin wird die Anwesenheit des organischen Lösungsmittels hervorgehoben. So wird bei der Cyclisierung des Diisopropanolamins der Formel HN-(CH₂-CH(OH)CH₃)₂ mit Raney-Nickel in Gegenwart von Toluol eine Selektivität von 81-82 %, bei Anwesenheit von Wasser anstelle von Toluol dagegen nur eine Selektivität von 72% zum cis-2,6-Dimethylpiperazin erreicht.

Aus der US-A-2,911,407 ist ein weiteres Verfahren zur Herstellung von 2,6-Dimethylpiperazin mit Umsätzen in der Größenordnung von 70% beschrieben. Hierzu wird Di-(2-hydroxypropyl)-amin mit Ammoniak in Gegenwart eines Nickel- oder Kobalt-haltigen Hydrier/Dehydrierkatalysators unter Druck umgesetzt, wobei mindestens 1 Mol Ammoniak pro Mol Di-(2-hydroxypropyl)-amin verwendet wird. Als Katalysator wird bevorzugt Raney-Nickel oder Raney-Cobalt eingesetzt. Es werden in der US-A-2,911,407 keinerlei Angaben darüber gemacht, ob und in welchen Anteilen das isolierte 2,6-Dimethylpiperazin die beiden cis- und trans-Stereoisomere enthält. Auch eine Aufspaltung des 2,6-Dimethylpiperazins in die beiden Stereoisomere wird nicht vorgenommen.

Aus der GB-A-902,570 ist ferner ein Verfahren zur Herstellung von C-Alkylsubstituierten Piperazinen aus Di-(2-hydroxyalkyl)aminen mit mindestens einer sekundären Hydroxylgruppe bekannt. Gemäß Beschreibung und Beispielen der GB-A-902,570 werden diese Di-(2-hydroxyalkyl)amine wie z.B. Diisopropanolamin in Form des Di-(2-hydroxypropyl)amins, 1-(2-Hydroxyethylamino)-2-propanol oder 3,3-Imino-di-2-butanol immer in Reinform und nicht als Mischungen verschiedener Isomere eingesetzt. Der bei der Reaktion verwendete Katalysator muß mindestens ein Metall oder Metalloxid aus der Gruppe Nickel, Kupfer und Kobalt enthalten. Zusätzlich kann er geringe Mengen Chromoxid, Molybdänoxid, Manganoxid, Thoriumoxid oder deren Mischungen als Promotoren aufweisen. Durch Umsetzung von Di-(2-hydroxypropyl)amin mit Ammoniak und Wasserstoff in Gegenwart eines Hydrierkatalysators aus Nickel, Kupfer und Chromoxiden wird 2,6-Dimethylpiperazin erhalten, wobei der in Beispiel II erzielte Umsatz bei über 90 % und die Ausbeute an 2,6-Dimethylpiperazin bei 74.3% liegt. GB-A-902, 570 enthält jedoch wie die bereits genannte US-A-2,911,407 keine Angaben darüber, ob und in welchen Anteilen sich das isolierte 2,6-Dimethylpiperazin aus den beiden cis- und trans-Stereoisomeren zusammensetzt. Genausowenig wird eine Aufspaltung des 2,6-Dimethylpiperazins in die beiden Stereoisomere vorgenommen.

Die zuvor genannten Verfahren gehen bei der Synthese von 2,6-Dimethylpiperazin immer von Reinsubstanzen wie dem Di-(2-hydroxypropyl)-amin aus, deren Isolierung immer mit Aufwand verbunden ist. Zudem existieren nur geringe Kenntnisse und Möglichkeiten zur gezielten und selektiven Darstellung von cis-2,6-Dimethylpiperazin.

Die Aufgabe der vorliegenden Erfindung bestand somit darin, ein Verfahren bereitzustellen, welches mit leichter zugänglichen Ausgangsstoffen eine selektive Herstellung sowie reine Gewinnung des Stereoisomers cis-2,6-Dimethylpiperazin ermöglicht.

Gegenstand der Erfindung ist ein Verfahren zur Herstellung von cis-2,6-Dimethylpiperazin durch Umsetzung eines Diisopropanolamin-Gemisches, welches Verbindungen der Formel HN-(CH₂-CH(OH)CH₃)₂, HN-(CH(CH₃)-CH₂OH)₂ und HN(CH(CH₃)-CH₂OH)(CH₂-CH(OH)CH₃) enthält, oder von 1,2-Diaminopropan mit Ammoniak und Wasserstoff in Gegenwart eines Hydrierkatalysators.

Das im erfindungsgemäßen Verfahren eingesetzte Diisopropanolamin-Gemisch, welches Verbindungen der Formel HN-(CH₂-CH(OH)CH₃)₂, HN-(CH(CH₃)-CH₂OH)₂ und HN(CH(CH₃)-CH₂OH)(CH₂-CH(OH)CH₃) enthält, fällt beispielsweise bei der Umsetzung von Propylenoxid mit Ammoniak zu Propanolamin an. Das bei dieser Umsetzung gegebenenfalls je nach Reaktionsführung ebenfalls entstehende Monoisopropanolamin sowie Triisopropanolamin werden abgetrennt, so dass das resultierende und im erfindungsgemäßen Verfahren einzusetzende Diisopropanolamin-Gemisch kein Monoisopropanolamin und Triisopropanolamin mehr enthält. Bei den Verbindungen HN-(CH₂-CH(OH)CH₃)₂, HN-(CH(CH₃)-CH₂OH)₂ und HN(CH(CH₃)-CH₂OH)(CH₂-CH(OH)CH₃) handelt es sich um drei Isomere, wobei die Verbindungen HN-(CH₂-CH(OH)CH₃)₂ und HN-(CH(CH₃)-CH₂OH)₂ jeweils zwei optische Antipoden umfassen und die Verbindung HN(CH(CH₃)-CH₂OH)(CH₂-CH(OH)CH₃) vier optische Antipoden umfaßt. Bevorzugt enthält das eingesetzte Diisopropanolamin-Gemisch
- mindestens 85 Gew.%: HN-(CH₂-CH(OH)CH₃)₂
- 5-10 Gew.%: HN(CH(CH₃)-CH₂OH)(CH₂-CH(OH)CH₃) und
- 0,1-2 Gew.%: HN-(CH(CH₃)-CH₂OH)₂

Besonders bevorzugt enthält das eingesetzte Diisopropanolamin-Gemisch
- 86-94 Gew.%: HN-(CH₂-CH(OH)CH₃)₂
- 8-10 Gew.%: HN(CH(CH₃)-CH₂OH)(CH₂-CH(OH)CH₃) und
- 0,1-1 Gew.%: HN-(CH(CH₃)-CH₂OH)₂

Ganz besonders bevorzugt wird ein Diisopropanolamin-Gemisch eingesetzt, welches
- 87-89 Gew.%: HN-(CH₂-CH(OH)CH₃)₂
- 8-10 Gew.%: HN(CH(CH₃)-CH₂OH)(CH₂-CH(OH)CH₃) und
- 0,1-1 Gew.%: HN-(CH(CH₃)-CH₂OH)₂

Insbesondere ergeben die vorstehend für die jeweiligen Diisopropanolamin-Gemische genannten Gew.% in der Summe 97, bevorzugt 98, besonders bevorzugt 99 und insbesondere 100 Gew.%.

1,2-Diaminopropan ist käuflich erhältlich.

Es ist überraschend, daß das erfindungsgemäße Verfahren unter Einsatz dieses Gemisches einer großen Zahl von insgesamt 8 Diisopropanolamin-Isomeren mit ausgezeichneter Selektivität und gleichzeitig guter Ausbeute zum cis-2,6-Dimethylpiperazin gelingt.

Als Hydrierkatalysatoren können aus dem Stand der Technik bekannte Hydrierkatalysatoren eingesetzt werden. Bevorzugt werden handelsübliche Raney-Katalysatoren verwendet. Besonders bevorzugt sind dabei Raney-Nickel und Raney-Kobalt. Bewährt haben sich auch Katalysatoren, die mindestens ein Metall oder Metalloxid aus der Gruppe Nickel, Kupfer, Kobalt und Eisen aufweisen. Nickel- und Eisenhaltige Katalysatoren, insbesondere solche mit einem Nickelgehalt von minimal 60 Gew.% und einem Eisengehalt von maximal 40 Gew.%, wie z.B. Ni/Fe 85/15, Ni/Fe 70/30, Ni/Fe 66/6 oder Ni/Fe 68/6, sind besonders bevorzugt. Die eingesetzten Hydrierkatalysatoren können ungeträgert sein, aber auch auf herkömmliche Trägermaterialien wie Kieselgel, Silicagel, Al₂O₃ oder SiO₂ aufgebracht sein. Sie können ferner auch geringe Mengen Chromoxid, Molybdänoxid, Manganoxid, Thoriumoxid oder deren Mischungen als Promotoren enthalten. Beschrieben sind solche Katalysatoren beispielsweise in der DE-OS-40 26 351, DE-OS-27 13 374, DE-OS-27 13 373 und DE-OS- 35 37 247.

Pro Mol Diisopropanolamin-Gemisch bzw. 1,2-Diaminopropan werden etwa 5-150 g, bevorzugt 20-100 g und besonders bevorzugt 30-50 g Katalysator eingesetzt.

Ammoniak wird üblicherweise unter Druck in reiner, flüssiger Form in einer Menge von 100-200 ml (4-8 Mol), bevorzugt 110-160 ml (4.4-6.4 Mol) pro Mol Diisopropanolamin-Gemisch bzw. 1,2-Diaminopropan zugesetzt. Es ist aber auch möglich, wäßrige Ammoniak-Lösungen zu verwenden.

Wasserstoff wird mit einem Druck von 1-12 MPa, bevorzugt von 2,5-10 MPa zugegeben. Die Reaktion wird bei einer Temperatur von 100-250°C, bevorzugt 150 - 220°C und insbesondere 190-210°C über einen Zeitraum von 2 - 10, bevorzugt 2,5 - 5 Stunden durchgeführt.

Die Anwesenheit weiterer organischer Lösungsmittel im erfindungsgemäßen Verfahren ist nicht zwingend, aber möglich. Eingesetzt werden können aromatische Kohlenwasserstoffe wie Benzol, Toluol oder Xylol, aliphatische Kohlenwasserstoffe wie n-Hexan oder Cyclohexan, aliphatische Alkohole wie Methanol, Ethanol oder Isopropanol oder Ether wie Dioxan, Dibutylether oder Morpholin. Die Reaktion kann auch in Gegenwart von Wasser durchgeführt werden, wenn wäßrige Ammoniak-Lösung verwendet wird.

Die erfindungsgemäße Umsetzung führt unter reduktiver Aminierung/dehydrierender Cyclisierung des oben definierten Diisopropanolamin-Gemisches bzw. 1,2-Diaminopropan zu einem Reaktionsgemisch der verschiedenen Isomeren von cis/trans-2,6-Dimethylpiperazin und cis/trans-2,5-Dimethylpiperazin, welches mindestens 85 Gew.% cis-2,6-Dimethylpiperazin und maximal 15 Gew.% der übrigen Isomeren (trans-2,6-, cis-2,5- und trans-2,5-Dimethylpiperazin) enthält. Diese bereits hohe Selektivität zum cis-2,6-Dimethylpiperazin läßt sich noch weiter verbessern, wenn man das Gemisch der trans-2,6-, cis-2,5- und trans-2,5-Isomeren destilliert und anschließend mindestens einmal - wie unten beschrieben - umkristallisiert. Nach Destillation und zweifacher Umkristallisation kann 100%ig reines cis-2,6-Dimethylpiperazin erhalten werden. Die Gesamtausbeute des Verfahrens beträgt mindestens 60 %, bevorzugt mindestens 65 % cis-2,6-Dimethylpiperazin, bezogen auf das eingesetzte Diisopropanolamin-Gemisch.

Die Umkristallisation des destillierten Reaktionsgemischs wird unter Verwendung eines Gemisches aus einem oder mehreren aliphatischen Alkoholen und einem oder mehreren Kohlenwasserstoffen als Lösungsmittel durchgeführt. Als aliphatische Alkohole kommen beispielsweise Isopropanol, Isobutanol oder Isoamylalkohol in Frage; bevorzugt wird Isopropanol eingesetzt. Als Kohlenwasserstoffe haben sich z.B. Leichtbenzin oder Petrolether bewährt, bevorzugt wird Leichtbenzin eingesetzt. Alkohole und Kohlenwasserstoffe werden in einem Volumen-Verhältnis von (0.5 - 9.5):10, bevorzugt (0.5 - 6):10 besonders bevorzugt (0.5 - 2):10 verwendet. Besonders bevorzugt wird eine zweifache Umkristallisation aus einem Isopropanol/Leichtbenzin-Gemisch im Volumen-Verhältnis von 1:10 durchgeführt.

Bei Verwendung von 1,2-Diaminopropan als Edukt erhält man unter den angegebenen Verfahrensbedingungen in Gegenwart von Raney-Ni bei 200°C z.B. ein Dimethylpiperazin-Isomerengemisch mit 18.3 % cis-2,5-Dimethylpiperazin, 13.7 % trans-2,5-Dimethylpiperazin, 43.7 % cis-2,6-Dimethylpiperazin und 5.7 % trans-2,6-Dimethylpiperazin.

In den nachfolgenden Beispielen wird unter dem Begriff 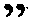 rohes Diisopropanolamin" immer ein Gemisch der Verbindungen HN-(CH₂-CH(OH)CH₃)₂, HN-(CH(CH₃)-CH₂OH)₂ und HN(CH(CH₃)-CH₂OH)(CH₂-CH(OH)CH₃) verstanden, welches bei der Reaktion von Propylenoxid und Ammoniak nach Abtrennung von Mono-isopropanolamin und Triisopropanolamin erhalten wird. Es enthält
- ca. 89 Gew.%: HN-(CH₂-CH(OH)CH₃)₂
- 8-10 Gew.%: HN(CH(CH₃)-CH₂OH)(CH₂-CH(OH)CH₃) und
- 0,1-1 Gew.%: HN-(CH(CH₃)-CH₂OH)₂

### Beispiel 1:

515 g rohes Diisopropanolamin (3.87 Mol) werden in 750 ml flüssigem Ammoniak (30 Mol) bei einem Wasserstoffdruck von 3 MPa 2 Stunden bei 200°C in Gegenwart von 100 g eines Ni/Fe 85/15 Katalysators (aus 85 Gew.% Ni und 15 Gew.% Fe) gerührt. Es stellt sich ein Enddruck von 15.9 MPa ein. Anschließend wird entspannt, das rohe Dimethylpiperazingemisch in 1,5 l Methanol gelöst, durch Filtration vom Katalysator befreit und die Lösung bei Normaldruck über eine 20 cm Füllkörperkolonne, die als Glasfüllkörper Raschigringe enthält,eingedampft.

Das gesamte erhaltene Reaktionsgemisch wird bei Normaldruck über eine Kolonne destilliert. Bei einem Siedepunkt von 150-153 °C erhält man 344 g Destillat (76.6% bezogen auf das eingesetzte rohe Diisopropanolamin) und 26 g Destillationsrückstand.

Eine 0.5 g Probe dieses Destillats wird zwecks Analyse der Zusammensetzung mit etwa 2 g Acetanhydrid bei Raumtemperatur vermischt und unter leichter Erwärmung eine Stunde stehengelassen. Diese acetylierte Probe wird dann unter Verwendung einer 20 m SE 30 Kapillarsäule gaschromatographisch analysiert und enthält:
2.01 % cis-2,5-Dimethylpiperazin
4.88 % trans-2,5-Dimethylpiperazin
84.94 % cis-2,6-Dimethylpiperazin
7.0 % trans-2,6-Dimethylpiperazin

Das gesamte Destillat wird in einem Gemisch aus 200 ml Isopropanol und 650 ml Leichtbenzin umkristallisiert, wobei erst langsam bis Raumtemperatur und dann bis 0°C abgekühlt wird. Nach Abfiltrieren und Trocknen des Kristallisats unter vermindertem Druck erhält man 235.5 g Produkt (52.5% bezogen aus das eingesetzte rohe Diisopropanolamin) mit einem Schmelzpunkt von 112.5 -113°C. Das Produkt wird, wie oben angegeben, gaschromatographisch analysiert und enthält:
0 % cis-2,5-Dimethylpiperazin
0 % trans-2,5-Dimethylpiperazin
99.6 % cis-2,6-Dimethylpiperazin
0.33 % trans-2,6-Dimethylpiperazin

Nochmaliges Umkristallisieren führt zu 178 g cis-2,6-Dimethylpiperazin 100%iger Reinheit (1.78 Mol) mit einem Schmelzpunkt von 113-114°C.

Die Gesamtausbeute beträgt somit 46 % bezogen auf das eingesetze rohe Diisopropanolamin. (1.78 Mol cis-2,6-Dimethylpiperazin bezogen auf 3.87 Mol eingesetztes rohes Diisopropanolamin).

### Beispiel 2:

980 g rohes Diisopropanolamin werden in 1,2 l flüssigem Ammoniak bei einem Wasserstoff-Druck von 3 MPa 3 Stunden bei 200°C in Gegenwart von 250 g Raney-Kobalt gerührt. Anschließend wird entspannt, das rohe Dimethylpiperazin-Gemisch in 1.5 l Methanol gelöst, durch Filtration vom Katalysator befreit und die Lösung bei Normaldruck über eine 20 cm Füllkörperkolonne, die als Glasfüllkörper Raschigringe enthält, eingedampft. Das Gaschromatgramm ergibt nach Acetylierung mit Acetanhydrid (wie in Beispiel 1 beschrieben) die folgenden Werte:
2.96 % cis-2,5-Dimethylpiperazin
3.22 % trans-2,5-Dimethylpiperazin
81.79 % cis-2,6-Dimethylpiperazin
6.31 % trans-2,6-Dimethylpiperazin

Der erhaltene Rückstand wird bei 145-163 °C unter Normaldruck über die zuvor genannte Kolonne destilliert. Man erhält 719 g Destillat mit einem Schmelzpunkt von 102°C. Eine Destillatprobe wird nach Acetylierung mit Acetanhydrid (wie in Beispiel 1 beschrieben) gaschromatographisch analysiert und enthält:
3.01 % cis-2,5-Dimethylpiperazin
3.25 % trans-2,5-Dimethylpiperazin
84.38 % cis-2,6-Dimethylpiperazin
4.78 % trans-2,6-Dimethylpiperazin

Das gesamte Destillat wird in einem Gemisch aus 150 ml Isopropanol und 1500 ml Leichtbenzin umkristallisiert, wobei erst langsam bis Raumtemperatur und dann bis 0°C abgekühlt wird. Nach Abfiltrieren und Trocknen des Kristallisats unter vermindertem Druck erhält man 569 g Produkt mit einem Schmelzpunkt von 110°C. Eine Kristallisatprobe wird nach Acetylierung gaschromatographisch analysiert und enthält:
0.91 % cis-2,5-Dimethylpiperazin
1.1 % trans-2,5-Dimethylpiperazin
95.08 % cis-2,6-Dimethylpiperazin
2.0 % trans-2,6-Dimethylpiperazin

Nochmaliges Umkristallisieren führt zu 502 g an 100%ig reinem cis-2,6-Dimethylpiperazin mit einem Schmelzpunkt von 113-114 °C. Aus der Mutterlauge kann noch weiteres cis-2,6-Dimethyl-piperazin isoliert werden, so daß eine Gesamtausbeute von 63% d. Theorie erreicht wird.

### Beispiel 3:

500 g rohes Diisopropanolamin werden mit 100 g Raney Nickel und 750 ml flüssigem Ammoniak bei 200°C unter einem Anfangsdruck von 3 MPa Wasserstoff gerührt. Der Enddruck beträgt 154 bar. Es wird entspannt, das rohe Produktgemisch in Methanol gelöst, durch Filtration vom Katalysator befreit und bei Normaldruck über eine 20 cm Füllkörperkolonne, die als Glasfüllkörper Raschigringe enthält, eingedampft. Eine Probe des Destillationsrückstandes wird nach Acetylierung gemäß Beispiel 1 gaschromatographisch analysiert und enthält:
2.55 % cis-2,5-Dimethylpiperazin
3.73 % trans-2,5-Dimethylpiperazin
83.81 % cis-2,6-Dimethylpiperazin
5.65 % trans-2,6-Dimethylpiperazin

Der gesamte Rückstand wird bei Normaldruck bei 150-166 °C über die oben genannte Kolonne destilliert. Man erhält 367 g Dimethylpiperazin-Destillat (85.6 % bezogen auf das eingesetzte rohe Diisopropanolamin) sowie 26 g Destillationsrückstand. Das Destillat enthält nach Acetylierung und gaschromatographischer Analyse:
2.61 % cis-2,5-Dimethylpiperazin
4.11 % trans-2,5-Dimethylpiperazin
84.49 % cis-2,6-Dimethylpiperazin
5.7 % trans-2,6-Dimethylpiperazin

Einmaliges Umkristallisieren des Destillates aus einem Gemisch von 260 ml Isopropanol und 1000 ml Leichtbenzin liefert 246 g Dimethylpiperazine, bei denen es sich nach gaschromatographischer Analyse zu 99.29 % um cis-2,6- und zu 0.7 % um trans-2,6-Dimethylpiperazin handelt. Nochmaliges Umkristallisieren liefert 220 g 100%ig reines cis-2,6-Dimethylpiperazin (entspricht 51.3% der Theorie bezogen auf das eingesetzte rohe Diisopropanolamin). Insgesamt werden nach Aufarbeitung der Mutterlauge 253,5 g cis-2,6-Dimethylpiperazin erhalten (59% der Theorie bezogen auf das rohe Dimethylpiperazin).

## Patentansprüche

1. Verfahren zur Herstellung von cis-2,6- Dimethylpiperazin durch Umsetzung eines Diisopropanolamin-Gemisches, welches Verbindungen der Formel HN-(CH₂-CH(OH)CH₃)₂, HN-(CH(CH₃)-CH₂OH)₂ und HN(CH(CH₃)-CH₂OH)(CH₂-CH(OH)CH₃) enthält, oder von 1,2-Diaminopropan mit Ammoniak und Wasserstoff in Gegenwart eines Hydrierkatalysators.

2. Verfahren nach Anspruch 1, dadurch gekennzeichnet, daß das eingesetzte Diisopropanolamin-Gemisch durch Umsetzung von Propylenoxid mit Ammoniak erhalten wird.

3. Verfahren nach Anspruch 1 oder 2, dadurch gekennzeichnet, daß als Hydrierkatalysatoren Raney-Nickel und Raney-Kobalt eingesetzt werden.

4. Verfahren nach Anspruch 1 oder 2, dadurch gekennzeichnet, daß die Hydrierkatalysatoren mindestens ein Metall oder Metalloxid aus der Gruppe Nickel, Kupfer, Kobalt und Eisen aufweisen

5. Verfahren nach Anspruch 4, dadurch gekennzeichnet, daß Nickel- und Eisenhaltige Katalysatoren eingesetzt werden, insbesondere mit einem Nickelgehalt von minimal 60 Gew.% und einem Eisengehalt von maximal 40 Gew.%.

6. Verfahren nach einem oder mehreren der Ansprüche 1-5, dadurch gekennzeichnet, daß pro Mol Diisopropanolamin-Gemisch bzw. 1,2-Diaminopropan 5-150 g, bevorzugt 20-100 g und besonders bevorzugt 30-50 g Katalysator eingesetzt werden.

7. Verfahren nach einem oder mehreren der Ansprüche 1-6, dadurch gekennzeichnet, daß Ammoniak in reiner, flüssiger Form in einer Menge von 100-200 ml (4-8 Mol), bevorzugt 110-160 ml (4.4-6.4 Mol) pro Mol Diisopropanolamin-Gemisch bzw. 1,2-Diaminopropan eingesetzt wird.

8. Verfahren nach einem oder mehreren der Ansprüche 1-7, dadurch gekennzeichnet, daß Wasserstoff mit einem Druck von 1-12 MPa, bevorzugt von 2,5-10 MPa zugegeben und die Reaktion bei einer Temperatur von 100-250°C, bevorzugt 150-220°C und insbesondere 190-210°C über einen Zeitraum von 2 - 10, bevorzugt 2,5 - 5 Stunden durchgeführt wird.

9. Verfahren nach einem oder mehreren der Ansprüche 1-8, dadurch gekennzeichnet, daß man im Anschluß an die Umsetzung des Diisopropanolamin-Gemisches oder von 1,2-Diaminopropan mit Ammoniak und Wasserstoff in Gegenwart des Hydrierkatalysators das resultierende Reaktionsgemisch destilliert und gegebenenfalls mindestens einmal umkristallisiert.

10. Verfahren nach Anspruch 9, dadurch gekennzeichnet, daß man die Umkristallisation unter Verwendung eines Gemisches aus einem oder mehreren aliphatischen Alkoholen, bevorzugt Isopropanol, Isobutanol oder Isoamylalkohol, und einem oder mehreren Kohlenwasserstoffen, bevorzugt Leichtbenzin oder Petrolether, als Lösungsmittel durchführt.

11. Verfahren nach einem oder mehreren der Ansprüche 1-10, dadurch gekennzeichnet, dass das eingesetzte Diisopropanolamin-Gemisch mindestens 85 Gew.%, bevorzugt 86-94 Gew.% und insbesondere 87-89 Gew.% HN-(CH₂-CH(OH)CH₃)₂, 5-10 Gew.%, bevorzugt 8-10 Gew.% HN(CH(CH₃)-CH₂OH)(CH₂-CH(OH)CH₃) und 0,1-2 Gew.%, bevorzugt 0,1-1Gew.% HN-(CH(CH₃)-CH₂OH)₂ enthält.

12. Verfahren nach Anspruch 11, dadurch gekennzeichnet, dass die Summe der Gew.% für HN-(CH₂-CH(OH)CH₃)₂, HN-(CH(CH₃)-CH₂OH)₂ und HN(CH(CH₃)-CH₂OH)(CH₂-CH(OH)CH₃) 97, bevorzugt 98, besonders bevorzugt 99 und insbesondere 100 Gew.% ergibt.

13. Verfahren nach einem oder mehreren der Ansprüche 1-12, dadurch gekennzeichnet, dass das eingesetzte Diisopropanolamin-Gemisch kein Monoisopropanolamin und kein Triisopropanolamin enthält.
